Europäisches Patentamt

European Patent Office

Office européen des brevets

(11) Veröffentlichungsnummer: **0 410 309 A1**

## (12) EUROPÄISCHE PATENTANMELDUNG

(21) Anmeldenummer: 90113888.3

(22) Anmeldetag: 19.07.90

(51) Int. Cl.5: **A61B 17/56**, A61F 5/02

(30) Priorität: 20.07.89 DE 3923995

(43) Veröffentlichungstag der Anmeldung:
30.01.91 Patentblatt 91/05

(84) Benannte Vertragsstaaten:
AT BE CH DE DK ES FR GB GR IT LI LU NL SE

(71) Anmelder: Biedermann, Lutz
Am Schäfersteig 8
D-7730 VS-Villingen(DE)

Anmelder: Harms, Jürgen, Prof. Dr.
Belchenweg 9

D-7517 Waldbronn-Reichenbach(DE)

(72) Erfinder: Biedermann, Lutz
Am Schäfersteig 8
D-7730 VS-Villingen(DE)
Erfinder: Harms, Jürgen, Prof. Dr.
Belchenweg 9
D-7517 Waldbronn-Reichenbach(DE)

(74) Vertreter: Prüfer, Lutz H., Dipl.-Phys.
Harthauser Strasse 25d
D-8000 München 90(DE)

(54) **Stabilisierungselement für Knochen.**

(57) Die Erfindung bezieht sich auf ein Stabilisierungselement für Knochen, welches insbesondere dazu dient, gebrochene bzw. angerissene Knochenteile während des Heilvorganges oder auf eine darüber hinausgehende Zeit in einer vorgegebenen Stellung zu halten. Das Stabilisierungselement weist eine mit Langlöchern (2 - $2^n$) versehene Leiste (1) auf, die an dem zu stabilisierenden Knochenteil (10) mit Hilfe von durch die Langlöcher ragenden Schrauben (4) befestigt wird. Das Problem bei einem derartigen Stabilisierungselement besteht darin, daß die Schrauben in der Regel nicht senkrecht zu der Leiste einschraubbar sind, weil das Einschrauben von der Richtung der Knochenteile abhängt. Damit auch bei einer Ausrichtung der Schraube, die nicht senkrecht zur Leiste ist, eine feste Verbindung erreicht wird, ist für jede Schraube ein Zwischenstück (7) vorgesehen, das mit einer Seite an der Leiste (1) aufliegt und an seiner anderen Seite mit einer kugelsegmentförmigen Ausnehmung (8) versehen ist, an der die kugelsegmentförmig geformte Unterseite (6) des Schraubenkopfes (5) anliegt.

FIG. 1

EP 0 410 309 A1

## STABILISIERUNGSELEMENT FÜR KNOCHEN

Solche Stabilisierungselemente dienen dazu, gebrochene bzw. angerissene Knochenteile während des Heilungsvorgangs oder auch für eine darüberhinausgehende Zeit in der Stellung zu halten, in der diese zusammenwachsen bzw. stabilisiert werden sollen.

Die Elemente bestehen aus einer Leiste, die mit einer Mehrzahl von Langlöchern versehen ist. Zwischen den Langlöchern können Stellen mit verringertem Querschnitt vorgesehen sein, um die Leiste dem gewünschten Verlauf des Knochens anzupassen. Die Befestigung der Leiste am Knochen erfolgt mit Schrauben, die in den Knochen eingedreht werden. Dabei tritt das Problem auf, daß die Schrauben in den seltensten Fällen senkrecht zur Leiste eingeschraubt werden können, da der Knochen unregelmäßig geformt ist und in der Richtung senkrecht zur Leiste selten einen ausreichenden Querschnitt aufweist.

Wird die Schraube aber in Richtung des größten Querschnitts des Knochens eingedreht, so liegt der Schraubenkopf nur einseitig im Langloch der Leiste auf und es findet keine ausreichende Fixierung zwischen Schraubenkopf und Leiste statt.

Der Erfindung liegt deshalb die Aufgabe zugrunde, ein Stabilisierungselement für Knochen zu schaffen, das auch bei sehr unregelmäßig geformten Knochen eine ausreichende Fixierung erlaubt.

Die Lösung dieser Aufgabe ist im Anspruch 1 angegeben.

Weiterbildungen der Erfindung sind Gegenstand der Unteransprüche.

In den Zeichnungen zeigt

Fig. 1 eine Draufsicht und eine Seitenansicht der Leiste mit eingesetzten Schrauben in Verbindung mit dem erfindungsgemäßen Zwischenstück.

In Fig. 2 ist eine Schraube und eine mögliche Ausführungsform für ein Zwischenstück dargestellt;

Die Fig. 3 bis 5 zeigen das Stabilisierungselement in Verbindung mit einem damit fixierten Halswirbel.

Die Figuren 6 bis 8 zeigen eine weitere mögliche Ausführungsform des Zwischenstücks 7, wobei die Figur 6 einen Schnitt durch das zerlegte Stabilisierungselement wiedergibt, während die Figur 7 einen Schnitt durch das zusammengesetzte Element und die Figur 8 eine Draufsicht der Figur 7 zeigt.

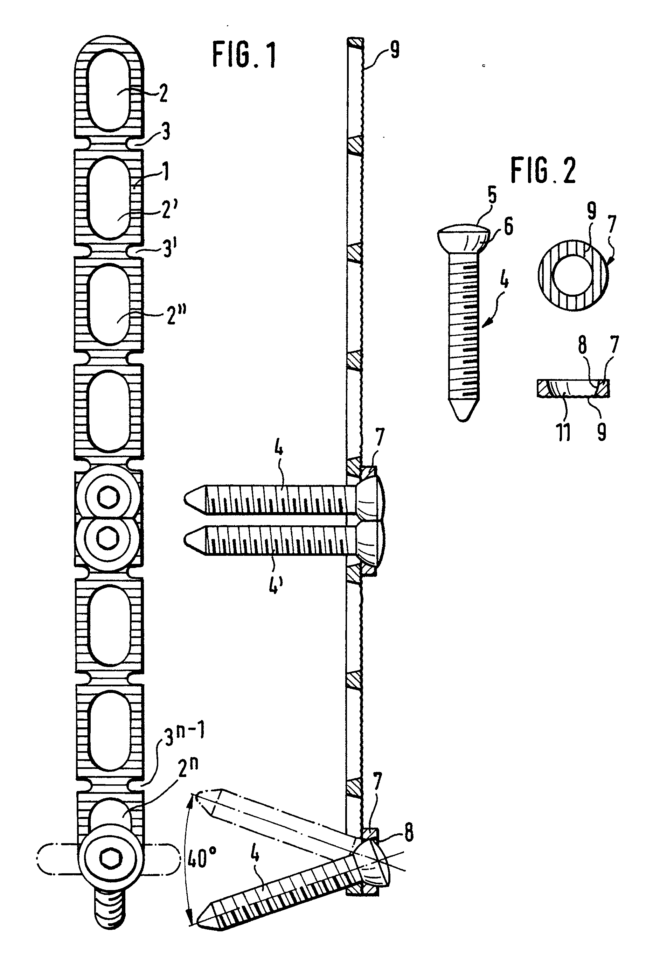

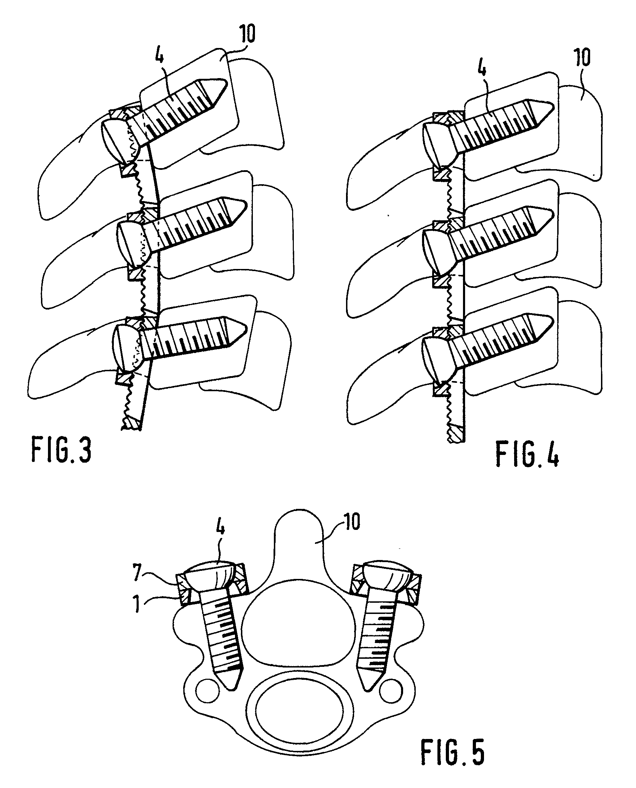

Die in Fig. 1 dargestellte Leiste 1 weist eine Reihe von Langlöchern 2 bis $2^n$ auf. Zwischen den Langlöchern kann die Leiste 1 eine Reihe von Stellen 3 bis $3^{n-1}$ mit verringertem Querschnitt aufweisen, was ein Anpassen der Leiste an die Form des Knochens ermöglicht (siehe Fig. 3).

Für jede einzusetzende Schraube ist ein Zwischenstück 7 vorgesehen, welches an der gewünschten Stelle auf die dem Knochen abgewandte Seite der Leiste 1 aufgesetzt wird und zur Aufnahme der Schraube 4 dient. Das Zwischenstück weist eine Bohrung 11 auf, die größer als der Schaft einer einzusetzenden Schraube ist, damit der Schaft der Schraube um einen möglichst großen Winkel schwenkbar ist. Aber sie ist nur so groß, daß der Schraubenkopf noch darin festgehalten wird. Auf ihrer der Leiste 1 abgewandten Oberfläche weist das Zwischenstück eine zur Bohrung 11 konzentrische kugelsegmentförmige Ausnehmung 8 auf.

Die in Fig. 2 einzeln dargestellte Schraube 4 hat an der Unterseite ihres Kopfes 5 eine kugelsegmentförmige Form 6, mit der sie in der kugelförmig gestalteten Ausnehmung 8 des Zwischenstücks 7 anliegt. Die Radien für Ausnehmung 8 und Form 6 entsprechen einander. Dadurch wird es möglich, die Schraube 4, wie im unteren Teil der Fig. 1 dargestellt, unter einem räumlichen Winkel von bis zu ca. $\pm 20°$ in das Knochengewebe einzuschrauben. Trotzdem liegt der gesamte Schraubkopf 5 am Zwischenstück 7 und über dieses an der Leiste 1 voll auf, wodurch eine wesentlich verbesserte Stabilisierung erzielt wird.

Die Lage der Schraube 4 gegenüber der Leiste 1 kann dadurch gesichert werden, daß, gemäß einer Weiterbildung der Erfindung, die Unterseite des Zwischenstücks 7 und die Oberseite der Leiste 1 mit einer Strukturierung versehen werden. Diese Strukturierung kann, wie in Fig. 1 gezeigt, als Riffelung 9 senkrecht zur Längsrichtung der Leiste 1 ausgebildet sein.

Damit wird eine Bewegung des Zwischenstücks 7 und damit der Schraube 4 nach dem Anziehen der Schraube positiv verhindert.

Im mittleren Teil der Fig. 1 ist angedeutet, daß gewünschtenfalls eine Verschiebung der Schraube innerhalb eines einzigen Langlochs 2 möglich ist.

In den Fig. 3 bis 5 ist ein Ausführungsbeispiel der Erfindung im montierten Zustand an einem Halswirbel 10 dargestellt.

In Fig. 3 ist die Leiste 1 an den Stellen 3, 3' etc. mit verringertem Querschnitt geknickt, um diese der Form des Wirbels 10 anzupassen.

Fig. 4 zeigt eine Ausführungsform mit gerader Leiste.

Die Fig. 5 stellt einen Schnitt durch einen Halswirbel 10 dar, der rechts und links mit je einem Element gemäß der Erfindung stabilisiert ist.

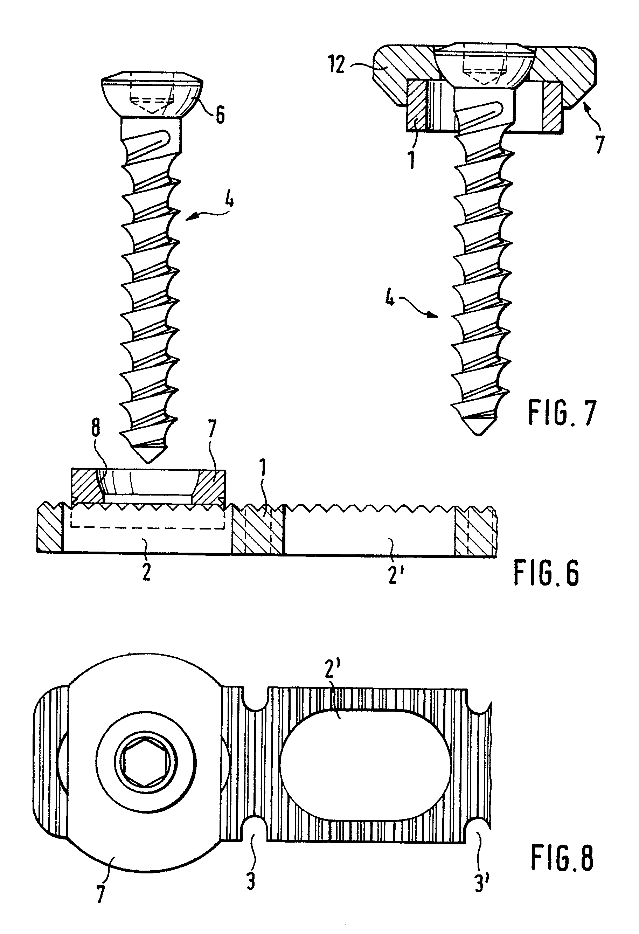

In den Figuren 6 bis 8 ist eine weitere Möglichkeit für die Ausbildung des Zwischenstücks 7 dargestellt.

Die Außenform des Zwischenstücks 7 ragt dabei an den Längsseiten der Leiste 1 über diese hinaus und ist mit einer Schulter 12 versehen, die die Leiste 1 beidseitig umfaßt. Dies bewirkt eine noch bessere Verankerung des Zwischenstücks 7 auf der Leiste 1 und verhindert gleichzeitig ein seitliches Verschieben des Zwischenstücks während der Befestigung, was eine Erleichterung des Operationsvorgangs bedeutet.

## Ansprüche

1. Stabilisierungselement für Knochen, bestehend aus einer mit Langlöchern ($2 - 2^n$) versehenen Leiste (1), die an dem zu stabilisierenden Knochenteil (10) mit Hilfe von durch die Langlöcher ragenden Schrauben (4) befestigt wird, gekennzeichenet durch ein Zwischenstück (7), das mit einer Seite an der Leiste (1) flach aufliegt und an seiner anderen Seite mit einer konkaven kugelsegmentförmigen Ausnehmnung (8) versehen ist, an der die konvex kugelsegmentförmig geformte Unterseite (6) des Schraubenkopfes (5) anliegt.

2. Stabilisierungselement nach Anspruch 1, dadurch gekennzeichnet, daß die Leiste (1) und das Zwischenstück (7) auf ihren zueinander gekehrten Seiten mit einer Strukturierung (9) versehen sind.

3. Stabilisierungselement nach Anspruch 2, dadurch gekennzeichnet, daß die Strukturierung aus einer senkrecht zur Längsrichtung der Leiste (1) verlaufenden Riffelung (9) besteht.

4. Stabilisierungselement nach Anspruch 1, dadurch gekennzeichnet, daß die Leiste (1) in Abständen Stellen ($3-3^{n-1}$) mit verringertem Querschnitt aufweist.

5. Stabilisierungselement nach Anspruch 1, dadurch gekennzeichnet, daß das Zwischenstück (7) zwei kugelförmige Ausnehmungen nebeneinander aufweist.

6. Stabilisierungselement nach Anspruch 1, dadurch gekennzeichnet, daß das Zwischenstück (7) eine rechteckige Außenform aufweist.

7. Stabilisierungselement nach Anspruch 1, dadurch gekennzeichnet, daß das Zwischenstück (7) eine runde Außenform aufweist.

8. Stabilisierungselement nach Anspruch 1, dadurch gekennzeichnet, daß die Langlöcher ($2-2^n$) der Leiste (1) an der dem Knochen (10) zugewandten Seite mit einer Facette versehen sind.

9. Stabilisierungselement nach Anspruch 1, dadurch gekennzeichnet, daß das Zwischenstück (7) eine Außenform aufweist, die über die Längsseiten der Leiste (1) hinausragt und mit einer Schulter (12) versehen ist, die die Leiste (1) beidseitig umgreift.

10. Stabilisierungselement nach Anspruch 9, dadurch gekennzeichnet, daß das Zwischenstück (7) an seiner der Leiste zugewandten Seite mit einer Facette und an seiner gegenüberliegenden Seite mit einer Abrundung versehen ist.

# FIG.1

# FIG.2

FIG.3

FIG.4

FIG.5

FIG.7

FIG.6

FIG.8

Europäisches
Patentamt

**EUROPÄISCHER
RECHERCHENBERICHT**

Nummer der Anmeldung

**EP 90 11 3888**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-2 933 659  (SULZER) <br> * Seite 4, Zeilen 29-35,4-6; Figur 1 * <br> — — — | 1,4-7,10 | A 61 B 17/56 <br> A 61 F 5/02 |
| Y | DE-C-2 512 46  (MINER) <br> * Figuren 1,2,3; Seite 1, Zeilen 46-50,54-59 * <br> — — — | 1-10 | |
| Y | JOURNAL OF BONE <br> & JOINT SURGERY, Band 70-A, Nr. 3, März 1988, Seiten 416-421, Boston, MA, US; R.R. RICHARDS et al.: "Shoulder arthrodesis using a pelvic-reconstruction plate" <br> * Figur 1; Seite 417, Zeilen 23-26 * <br> — — — | 1-10 | |
| A | FR-B-1 037 262  (STEENBRUGGHE) <br> * Figuren 1,2,3; Seite 1, Zeilen 23-24; Seite 2, Zeilen 3-6 * <br> — — — | 3,9,6 | |
| A | FR-A-2 612 070  (PRIVAT) <br> * Figur 4, Teile 3,4 * <br> — — — | | |
| A | FR-A-2 556 583  (INSERM) <br> — — — | | |
| A | DE-A-3 630 862  (MECRON) <br> — — — | | RECHERCHIERTE SACHGEBIETE (Int. Cl.5) |
| A | EP-A-0 196 206  (THACKRAY) <br> — — — — — | | A 61 B <br> A 61 F |

Der vorliegende Recherchenbericht wurde für alle Patentansprüche erstellt

| Recherchenort | Abschlußdatum der Recherche | Prüfer |
|---|---|---|
| Den Haag | 19 Oktober 90 | BARTON S.A. |